(19) Europäisches Patentamt
European Patent Office
Office européen des brevets

(11) Numéro de publication: **0 328 460**
**A2**

(12) **DEMANDE DE BREVET EUROPEEN**

(21) Numéro de dépôt: **89400385.4**

(22) Date de dépôt: **10.02.89**

(51) Int. Cl.[4]: **C 07 D 295/10**
A 61 K 31/55

(30) Priorité: **10.02.88 FR 8801565**

(43) Date de publication de la demande:
**16.08.89 Bulletin 89/33**

(84) Etats contractants désignés:
**AT BE CH DE ES FR GB GR IT LI LU NL SE**

(71) Demandeur: **LABORATOIRE L. LAFON**
**19 avenue du Professeur Cadiot**
**F-94700 Maisons Alfort Cédex (FR)**

(72) Inventeur: **Lafon, Louis**
**5, Rue de l'Alboni**
**F-75016 Paris (FR)**

(74) Mandataire: **Le Guen, Gérard et al**
**CABINET LAVOIX 2, place d'Estienne d'Orves**
**F-75441 Paris Cédex 09 (FR)**

(54) **1-(4-aminophényl)-2-hexaméthylèneiminopropanone, procédé de préparation et utilisation en thérapeutique.**

(57) La présente invention concerne la 1-(4-aminophényl)-2-hexaméthylèneiminopropanone et ses sels d'addition pharmaceutiquement acceptables.

Ces produits sont utiles en thérapeutique en raison de leurs effets antidépresseurs sur le SNC et de leurs propriétés immunologiques.

EP 0 328 460 A2

## Description

### 1-(4-aminophényl)-2-hexaméthylèneiminopropanone, procédé de préparation et utilisation en thérapeutique

La présente invention concerne la 1-(4-aminophényl)-2-hexaméthylèneimino-propanone et ses sels d'addition pharmaceutiquement acceptables. Elle concerne également un procédé de préparation de ces produits et leur utilisation en thérapeutique.

On sait que l'on a déjà préconisé dans le passé des dérivés de 1-(aminophényl)-2-aminopropanone de formule

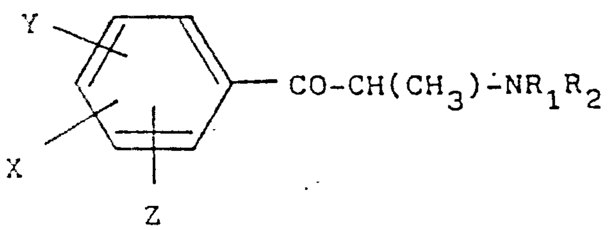

(I₀)

dans laquelle
X est $NH_2$
Y est un atome d'hydrogène ou d'halogène,
Z est un atome d'hydrogène ou d'halogène,
$R_1$ est un groupe alkyle en $C_1$-$C_4$ ou un groupe cycloalkyle en $C_3$-$C_6$,
$R_2$ est l'atome d'hydrogène ou un groupe alkyle en $C_1$-$C_4$,
$R_1$ et $R_2$, considérés ensemble, peuvent former avec l'atome d'azote auquel ils sont liés un groupe N-hétérocyclique de 5 à 7 sommets, pouvant compter un second hétéroatome choisi parmi N, O et S et pouvant être substitué, ledit groupe hétérocyclique $NR_1R_2$ étant choisi parmi l'ensemble comprenant les groupes pyrrolidino, morpholino, thiomorpholino, pipéridino, hexaméthylèneimino, pipérazino, 4-méthylpipérazino, 4-(2-hydroxyéthyl)-pipérazino, 4-phénylpipérazino, 4-(p-chlorophényl)-pipérazino ;
et leurs sels d'addition, en tant qu'agents antid presseurs du système nerveux central (SNC). On sait également que, parmi les composés de formule $I_0$ ci-dessus, quelques produits seulement présentent en outre des propriétés cardiovasculaires et/ou immuno-logiques bénéfiques. Voir à cet effet le brevet EP-B-0 174 242.

Suivant l'invention on propose de nouveaux produits appartenant à la famille des dérivés de 1-(aminophényl)-2-aminopropanone, à savoir la 1-(4-aminophényl)-2-hexaméthylène-iminopropanone et ses sels d'addition.

On propose également un procédé de préparation de ladite 1-(4-aminophényl)-2-hexaméthylèneiminopropanone, cette base libre et ses sels d'addition pharmaceutiquement acceptables étant particulièrement utiles en thérapeutique en raison de leurs effets antidépresseurs, d'une part, et de leurs effets immuno-modulateurs, d'autre part.

La 1-(4-aminophényl)-2-hexaméthylèneiminopropanone est certes incluse dans la formule générale du brevet EP-B-0 174 242 bien qu'elle ne soit pas spécifiquement exemplifiée. Elle présente des effets antidépresseurs comme l'ensemble des composés décrits dans EP-B-0 174 242, mais se distingue de ses analogues les plus proches du point de vue de la structure par le fait qu'elle possède des effets immuno-modulateurs bénéfiques permettant son utilisation en tant qu'agent immuno-stimulant. Voir les résultats des essais comparatifs donnés ci-après.

Les nouveaux dérivés de 1-(aminophényl)-2-aminopropanone suivant l'invention sont caractérisés en ce qu'ils sont choisi parmi l'ensemble comprenant:

a) la 1-(4-aminophényl)-2-hexaméthylèneiminopropanone de formule développée

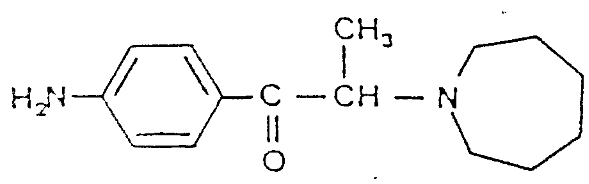

(I)

b) de ses sels d'addition pharmaceutiquement acceptables.

Par sels d'addition pharmaceutiquement acceptables, on entend ici les sels qui donnent les propriétés biologiques de la base libre sans avoir d'effets indésirables. Ces sels peuvent être les sels obtenus par réaction de la base libre de formule I avec un acide minéral ou organique, ou être des sels d'ammonium. Parmi les acides utilisables pour salifier la base libre de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition avec un acide, tels que notamment le dichlorhydrate, sont préférés aux sels d'ammonium.

Le composé de formule I peut être préparé selon une méthode connue en soi par application de mécanismes réactionnels classiques. Il peut notamment être synthétisé suivant l'une des méthodes opératoires appropriées décrites dans EP-B-0 174 242.

Le procédé que l'on préconise ici consiste à hydrolyser le dérivé 4-acétamido correspondant en milieu acide fort. Ce procédé est caractérisé en ce que l'on fait réagir 0, 10 à 0,20 mole de 1-(4-acétylaminophényl)-2-hexaméthylèneiminopropanone avec 100 à 300 ml d'acide HCl 4 N, pendant au moins 0,5 h à la température de reflux du milieu réactionnel.

La 1-(4-acétylaminophényl)-2-hexaméthylèneiminopropanone peut elle-même être obtenue par réaction en milieu aqueux de l'α-chloro p-acétamido propiophénone sur un excès d'hexaméthylèneimine.

Le composé de formule I et ses sels d'addition pharmaceutiquement acceptables présentant des propriétés thérapeutiques bénéfiques. Ils agissent en tant que moyens antidépresseurs; ils présentent en outre des effets stimulants du SNC ce qui n'est pas le cas de tous les produits décrits dans EP-B-0138 714.

Mais l'intérêt du composé de formule I et de ses sels d'addition pharmaceutiquement acceptables réside principalement dans le fait qu'ils présentent, outre des effets stimulants du SNC, des effets immuno-stimulants.

De nombreuses études ont montré que des stimulis psychologiques tels que le stress et la dépression peuvent avoir une influence profonde dans un grand nombre de processus physiologiques. Ainsi l'exposition à des situations créant un stress s'accompagne d'une diminution des fonctions immunologiques, ce qui conduit à des maladies infectieuses. L'existence d'un tel lien entre l'esprit et l'immunité et vice versa constitue le fondement de l'intérêt thérapeutique des composés selon l'invention qui possèdent à la fois des propriétés antidépressives et stimulante sur le système nerveux central et des effets immunostimulants. Ils trouvent donc un intérêt particulier dans le traitement des états dépressifs et des situations induisant un stress associés à des maladies infectieuses.

Selon l'invention on préconise une composition thérapeutique qui est caractérisée en ce qu'elle renferme, en association avec un excipient physiologiquement acceptable, au moins un dérivé choisi parmi le composé de formule I et ses sels d'addition pharmaceutiquement acceptables.

Bien entendu, dans une telle composition le principe actif, à savoir le composé de formule I ou l'un de ses sels pharmaceutiquement acceptables, intervient en quantité pharaceutiquement efficace.

Suivant l'invention on préconise l'utilisation d'une substance choisie parmi l'ensemble comprenant (i) 1-(4-aminophényl)-2-hexaméthylèneiminopropane et (ii) ses sels d'addition pharmaceutiquement acceptables, pour l'obtention d'un médicament antidépresseur du SNC destiné à une utilisation en thérapeutique humaine vis-à-vis des dépressions et des états dépressifs.

Suivant l'invention on préconise également l'utilisation d'une substance choisie parmi l'ensemble comprenant la 1-(4-aminophényl)-2-hexaméthylèneiminopropanone, et ses sels d'addition pharmaceutiquement acceptables, pour l'obtention d'un médicament immuno-stimulant destiné à une utilisation en thérapeutique humaine dans le cas où une immuno-stimulation est requise.

D'autres avantages et caractéristiques de l'invention seront mieux compris à la lecture qui va suivre d'un exemple de préparation et de résultats d'essais pharmacologiques, l'ensemble de ces éléments n'étant nullement limitatif mais donné à titre d'illustration.

EXEMPLE

Préparation du dichlorhydrate de 1-(4-aminophényl)-2-hexaméthylèneiminopropanone

(N° de Code CRL 41 405)

a) Préparation de l'α-chloro p-acétamido propiophénone

Au sein d'un mélange de 69,2 g (0,50 mole) d'acétanilide et de 205 g (1,50 mole) de chlorure d'aluminium dans 525 ml de sulfure de carbone, on coule en 1 h 30 : 118 g (0,93 mole) de chlorure d'α-chloropropionyle et on chauffe au reflux 1 h. On décante le surnageant, et on hydrolyse le résidu par 1900 ml d'eau glacée et 385 ml d'acide chlorhydrique 4N.

Le précipité est isolé par filtration, repris par du benzène que l'on distille azéotropiquement au Dean-Stark. Après traitement de la solution chaude au noir CXA et refroidissement, on isole : 102,4 g d'une poudre légèrement beige.

$F_{inst.}$ (Kofler) = 120° C.

Rendement = 90,82 %.

b) Préparation de la 1-(4-acétylaminophényl)-2-hexaméthylèneiminopropanone, chlorhydrate.

On agite pendant 3 heures à la température ambiante : 56 g (0,248 mole) du produit obtenue en a) et 280 ml (2,48 mole) d'hexaméthylèneimine dans 300 ml d'eau. On dilue le milieu réactionnel par 500 ml d'eau, on extrait l'insoluble par de l'acétate d'éthyle que l'on lave abondamment par de l'eau. La phase organique est séchée sur sulfate de sodium sec puis traitée par de l'éthanol chlorhydrique.

Le précipité obtenu est purifié par deux cristallisations successives dans l'éthanol absolu et l'eau pour donner : 59 g d'une poudre blanche soluble dans l'eau à 3 %.

F ≃ 210° C (d)
Rendement = 73,3 %
Rendement total = 66,6 %.

c) Préparation du dichlorhydrate de 1-(4-aminophényl)-2-hexaméthylèneiminopropanone

On chauffe au reflux pendant 1 h une solution de 52 g (0,16 mole) de chlorhydrate de 1-(4-acétylaminophényl)-2-hexaméthylèneiminopropanone dans 250 ml d'acide HCl 4 N. On traite ensuite le milieu réactionnel avec du noir de carbone ("noir 3S") et, après filtration, évapore le filtrat sous pression réduite pour l'amener à siccité. Le résidu huileux ainsi obtenu est repris avec du benzène que l'on distille azéotropiquement au moyen d'un Dean-Stark, pour donner après filtration du précipité formé 51,5 g de produit sous la forme d'une poudre blanche à reflets verts, soluble dans l'eau à 400 g/l.

$F_{inst}$ (Kofler) = environ 160°C
Rendement = proche de 100 %.

On a résumé ci-après les résultats des essais qui ont été entrepris avec le CRL 41 405 suivant l'invention.

A. ETUDE IMMUNOLOGIQUE

L'étude immunologique a été réalisée suivant plusieurs protocoles pour apprécier, par des essais comparatifs, les éventuelles propriétés immuno-modulatrices du CRL 41 405 et de ses analogues.

On a notamment utilisé le test dit des cellules formant des plages de lyse par A.J. Cunningham et al. ("Further improvements in the plaque technique for detecting single antibody forming cells"), Immunology 14, pages 599-601, (1968), d'une part, et le test dit de l'intensité de l'hypersensibilité retardée aux globules rouges de mouton décrit par T.E. Miller et al. ("Immunopotentiation with BCG II modulation of the response to sheep blood cells"), Journal of the National Cancer Institute 51 (N° 5), pages 1669-1676, (1973), d'autre part.

1°) Le test des cellules formant plage de lyse (ou PFC IgM).

Ce test explore l'immunité humorale. Quatre jours après immunisation par un antigène T dépendant (ici des globules rouges de mouton), on dénombre les cellules spléniques exprimant une réponse directe anticorps IgM. Les souris utilisées à cet effet sont des souris conventionnelles $OF_1$ femelles, exemples d'organismes pathogènes spécifiques, pesant de 20 à 30 g, et, réparties suivant un lot témoin de 14 animaux et des lots de 7 animaux chacun par dose et par produit à étudier administré par voie orale le même jour que l'antigène. Un indice d'activité I est calculé pour chaque dose de produit selon la relation :

$$I = \frac{\text{moyenne de lyses par rate des souris traitées}}{\text{moyenne de lyses par rate des souris témoins}}$$

Ce test est réalisé au moins deux fois pour chaque dose (0,001 ; 0,01 . 0,1 ; 10 ; et 100 mg/kg) de produit à tester, et une étude statistique est entreprise au moyen du test t de Student après analyse de variance sur le nombre de lyses par rate.

On observe que le CRL 41 405 présente à partir de la dose de 1 mg/kg une activité et que l'indice d'activité augmente avec la dose pour atteindre la valeur I = 1,58 à la dose de 100 mg/kg P.O.

A titre de comparaison, le dichlorhydrate de la 1-(4-aminophényl)-2-pipéridinopropanone (CRL 41 241) décrit dans EP-B0 174 242 ne donne de réaction qu'à la dose de 100 mg/kg.

2°) Le test de l'intensité de l'hypersensibilité retardée aux globules rouges de mouton.

C'est une technique d'exploration de l'immunité cellulaire. Le composé à étudier est administré soit per os, s'il est insoluble, soit (comme c'est le cas ici) par voie sous-cutanée dans le coussinet de la patte, lorsqu'il est soluble dans l'eau, l'administration dudit composé intervenant trois jours avant immunisation (par administration sous-cutanée de globules rouges de mouton dans le coussinet plantaire) de souris conventionneles $OF_1$ femelles réparties suivant un lot témoin de 10 animaux et des lots de 5 animaux chacun par dose et par produit à étudier.

Ce test est réalisé au moins deux fois pour chaque dose (0,001 ; 0,01 ; 0,1 ; 10 et 100 mg/kg) de produit à tester et une étude statistique est entreprise comme indiqué ci-dessus.

On mesure la variation de l'épaisseur du coussinet plantaire afin de connaître le pourcentage d'augmentation de l'épaisseur du coussinet plantaire. On calcule ensuite un indice d'activité suivant la relation
$I_{HSR} = E/E_0$
où
E = moyenne du pourcentage d'augmentation de l'épaisseur du coussinet plantaire des souris traitées ; et,
$E_0$ = moyenne du pourcentage d'augmentation de l'épaisseur du coussinet plantaire des souris témoins.

On constate que le CRL 41 405 suivant l'invention donne une réponse positive à toutes les doses utilisées pour atteindre la valeur $I_{HSR}$ =1,77 à la dose de 100 mg/kg S.C.

Tous ces résultats mettent en évidence que le CRL 41 405 (i) est un produit immuno-modulateur et (ii) agit plus précisément en tant que substance immuno-stimulante.

B. ETUDE NEUROPSYCHOPHARMACOLOGIQUE

Au cours de l'étude des propriétés neuropsychopharmacologiques, le CRL 41 405 en solution dans de l'eau distillée a été administré par voie intrapéritonéale sous un volume de 20 ml/kg chez la souris mâle et de 5 ml/kg chez le rat mâle.

Le pH de la solution aqueuse devant être injectée varie en fonction de la concentration en CRL 41 405, comme indiqué dans le tableau ci-après.

TABLEAU

| Concentration du CRL 41 405 dans la solution aqueuse d'administration | pH de la solution aqueuse d'administration |
|---|---|
| 50 g/l | 1,5 |
| 1,6 g/l | 2,5 |
| 0,8 g/l | 3,5 |
| 0,2 g/l | 4,5 |
| 0,05 g/l | 5,5 |

I. TOXICITE

Chez la souris mâle, la DL-0 (dose maximale non mortelle) par voie intrapéritonéale du CRL 41 405 est supérieure à 64 mg/kg, la DL-60 (dose létale pour 60 % des animaux traités) est de l'ordre de 128 mg/kg et la DL-100 (dose minimale mortelle pour tous les animaux traités) est inférieure à 256 mg/kg.

II. COMPORTEMENT GLOBAL ET REACTIVITES

Des lots de trois animaux sont observés avant, puis 0,25 h, 0,50 h, 1H, 2h, 3h et 24 h après l'administration de CRL 41 405. On constate :

1°) Chez la souris

à la dose de 0,5 mg/kg :
- un comportement et des réactivités sensiblement comparables à ceux du lot témoin ;

aux doses de 2mg/kg et 8 mg/kg :
- une excitation, l'intensité maximale se manifestant 2 h après administration du CRL 41 405;

à la dose de 16 mg/kg :
- une excitation, l'intensité maximale se manifestant 2 à 2,5 h après administration du CRL 41 405 ;
- une exophtalmie ;

à la dose de 32 mg/kg :
- une exophtalmie pendant 1 h,
- une excitation d'une durée de 2 à 2,5 h, accompagnée de stéréotypies pendant 0,5 h,
- une augmentation de la réaction de peur et une augmentation de la réactivité au toucher,
- une hypothermie pendant 2 h (- 2° C, 30 minutes après administration du CRL 41 405); et,
- une mydriase modérée pendant 3 h ;

à la dose de 64 mg/kg :
- une excitation pendant 5 h,
- une exophtalmie,

- des convulsions,
- pas de mortalité.

2°) Chez le rat :

aux doses de 0,25 mg/kg, 1 mg/kg et 4 mg/kg :
- un comportement, des réactivités, une variation de la température rectale et du diamètre pupillaire sensiblement comparables à ceux du lot témoin ;

à la dose de 16 mg/kg :
- une excitation pendant 1 h,
- des mouvements stéréotypés,
- une mydriase pendant 2 h.

III. AUTRES ESSAIS

Les essais réalisés suivant les modalités opératoires décrites dans EP-B-0 174 242 (i.e. interaction avec l'apomorphine, interaction avec l'amphétamine, interaction avec la réserpine, interaction avec l'oxotrémorine, action sur le test des quatre plaques, la traction et l'électrochoc, action sur la motilité spontanée, action sur l'aggressivité intergroupes, action vis-à-vis de la motilité réduite par habituation à l'enceinte, action vis-à-vis de la motilité réduite par agression hypoxique, action vis-à-vis de l'anoxie asphyxique, interaction avec le barbital, action sur le "désespoir comportemental"), ont permis de démontrer que le CRL 41 405 présente dans son profil neuropsychopharmacologique des effets:
- antidépresseurs, objectivés par l'antagonisme des hypothermies induites par l'apomorphine, la réserpine ou l'oxotrémorine, d'une part, et par la diminution de la durée de l'immobilité dite de désespoir, d'autre part; et,
- stimulants du SNC tant chez le rat (présence de mouvements stéréotypés, potentialisation des stéréotypies amphétaminiques) que chez la souris (antagonisme du sommeil barbiturique, reprise de l'activité motrice chez l'animal habitué à son enceinte, amélioration de la récupération motrice après anoxie hypobare et augmentation modérée de la motilité spontanée).

Le CRL 41 405 présente en outre des signes de stimulation alpha-adrénergique périphérique (mydriase, antagonisme modéré ou ptôsis réserpinique et antagonisme des tremblements induits par l'oxotrémorine) ; toutefois selon ces tests le CRL 41 405 apparaît comme étant un moyen stimulant $\alpha$-adrénergique très faible.

**Revendications**

1. 1-(4-aminophényl)-2-hexaméthylèneiminopropanone et ses sels d'addition pharmaceutiquement acceptables.
2. Dichlorhydrate de 1-(4-aminophényl)-2-hexaméthylèneiminopropanone.
3. Composition thérapeutique comprenant à titre de principe actif un composé selon la revendication 1 ou la revendication 2.
4. Utilisation d'une substance choisie parmi l'ensemble comprenant (i) 1-(4-aminophényl)-2-hexaméthylèneiminopropanone et (ii) ses sels d'addition pharmaceutiquement acceptables, pour l'obtention d'un médicament antidépresseur du SNC destiné à une utilisation en thérapeutique humaine vis-à-vis des dépressions et des états dépressifs.
5. Utilisation d'une substance choisie parmi l'ensemble comprenant la 1-(4-aminophényl)-2-hexaméthylèneiminopropanone et ses sels d'addition pharmaceutiquement acceptables, pour l'obtention d'un médicament immuno-stimulant destiné à une utilisation en thérapeutique humaine dans le cas où une immuno-stimulation est requise.
6. Procédé de préparation de la 1-(4-aminophényl)-2-hexaméthylèneiminopropanone et de ses sels d'addition, ledit procédé étant caractérisé en ce que l'on effectue l'hydrolyse en milieu acide du dérivé 4-acétylaminophényle correspondant.
7. Procédé de préparation d'une composition thérapeutique, comprenant le mélange de la 1-(4-aminophényl)-2-hexaméthylèneiminopropanone ou d'un de ses sels d'addition avec un véhicule pharmaceutiquement acceptable.